(19) Europäisches Patentamt
European Patent Office
Office européen des brevets

(11) Veröffentlichungsnummer: **0 502 595 A2**

(12) # EUROPÄISCHE PATENTANMELDUNG

(21) Anmeldenummer: **92250052.5**

(22) Anmeldetag: **05.03.92**

(51) Int. Cl.5: **C07C 331/28**, A61K 43/00, A61K 49/02, C07C 233/51, C07C 237/20, C07D 309/12, C07D 207/335, C07C 233/13, C07C 323/32, C07C 317/44, C07C 331/14

(30) Priorität: **07.03.91 DE 4107571**

(43) Veröffentlichungstag der Anmeldung:
**09.09.92 Patentblatt 92/37**

(84) Benannte Vertragsstaaten:
**AT BE CH DE DK ES FR GB GR IT LI LU NL SE**

(71) Anmelder: **INSTITUT FÜR DIAGNOSTIKFORSCHUNG GmbH AN DER FREIEN UNIVERSITÄT BERLIN**
**Klinikum Rudolf Virchow, Spandauer Damm 130**
**W-1000 Berlin 19(DE)**

(72) Erfinder: **Neumeier, Reinhard, Dr.**
**Gabrielenstrasse 63**
**W-1000 Berlin 27(DE)**
Erfinder: **Kramp, Wolfgang, Dr.**
**Damwildsteig 41a**
**W-1000 Berlin 27(DE)**
Erfinder: **Rohlfs, Gerhard, Dr.**
**Sandhausener Strasse 24**
**W-1000 Berlin 27(DE)**

(54) **Amid-Chelate, deren Metallkomplexe sowie ihre Verwendung in Diagnostik und Therapie.**

(57) Die Erfindung betrifft Verbindungen der allgemeinen Formel I

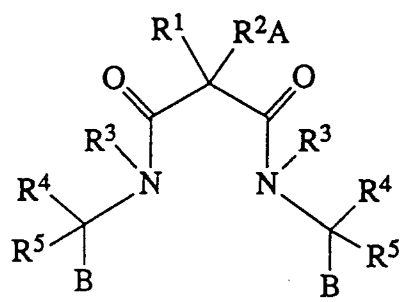

(I)

worin A eine funktionelle Gruppe beinhaltet, die für die Kopplung an sich selektiv anreichernde Verbindungen geeignet ist und $R^1$, $R^2$, $R^3$, $R^4$ und $R^5$ unterschiedliche Bedeutung haben können. B ist eine Gruppe, die für die koordinative Bindung von Metallionen geeignet ist. Die neuen Verbindungen dienen zur Komplexierung von radioaktiven Metallionen, insbesondere Rhenium- und Technetium-Isotopen, und werden in der medizinischen Diagnostik und Therapie eingesetzt.

EP 0 502 595 A2

Seit längerer Zeit werden radioaktive Metallionen, meist gebunden an einen Komplexbildner, für die in vivo-Diagnostik verwendet. Hiervon ist Technetium-99m (Tc-99m) aufgrund seiner für diese Zwecke nahezu idealen physikalischen Eigenschaften- gute Absorption der Strahlung in entsprechenden Detektionsgeräten (Gammakamera, SPECT-Geräte) gegenüber einer geringen Absorption im menschlichen Organismus und leichte Verfügbarkeit über einen Molybdän/Technetium-Generator - das am häufigsten in der klinischen Nuklearmedizin verwendete Radio -nuklid. Seine kurze Halbwertszeit von 6,02 h garantiert eine nur geringe Belastung des Patienten mit Gammastrahlung, zumal auch das Tochternuklid Technetium-99 nur eine vernachlässigbare Reststrahlung besitzt. Ein Nachteil des Technetiums ist allerdings seine komplizierte und noch nicht vollständig bekannte Komplex-Chemie. Technetium kann in einer Reihe von Oxidationsstufen (+7 bis -1) vorliegen, die die pharmakologischen Eigenschaften durch Veränderung der Ladung eines Komplexes stark verändern können. Es ist deswegen nötig, Komplexe zu synthetisieren, die das Technetium in einer definierten Oxidationstufe binden und Redox-Reaktionen, die zu einer Redistribution des Pharmakons führen könnten, verhindern. Eine Reihe solcher Tc-99m-Komplexbildner sind bereits bekannt und werden klinisch angewendet. Bei neutralen Komplexen handelt es sich vielfach um Systeme, in denen das Tc-99m zwischen 2-4 Stickstoffatomen und 0-2 Schwefelatomen gebunden wird ($N_2S_2$,- $N_3S$- und Propylenaminoximkomplexe). Vielfach ist jedoch die ungenügende Stabilität dieser Tc-99m-Komplexe ein wesentlicher Nachteil (Hung, J.C. et al.; J. Nucl. Med. 29: 1568, 1988). In der klinischen Anwendung muß deswegen z.B. HMPAO (Hexamethyl-propylenaminoxim) kurz nach seiner Markierung mit Pertechnetat appliziert werden, damit der Anteil an Zerfallsprodukten, die die diagnostische Aussagekraft vermindern, nicht zu hoch wird. Eine Kopplung dieser Chelate bzw. Chelatbildner an andere, sich selektiv in Krankheitsherden anreichernde Substanzen ist nicht möglich. Daher verteilen sich die meisten der erwähnten Komplexe entsprechend der Durchblutung und/oder metabolischen Aktivität eines Organs (z.B. Europ. Patent Appl. 0 194 843), so daß z.B. nekrotische oder ischämische Regionen nach Infarkt oder Schlaganfall in einem Szintigramm dargestellt werden können.

Für eine erfolgreiche Diagnostik von Tumoren, neurologischen Erkrankungen oder Herz-Kreislauferkrankungen sind jedoch Substanzen vielversprechender, die molekulare Veränderungen der erkrankten Gewebe darstellen können, indem sie spezifisch an diese erkrankten Gewebe binden bzw. in deren Stoffwechsel eingeschleust werden. Die Erkenntnisse der biologischen und biochemischen Grundlagenforschung erlauben die Auswahl einer Reihe von Substanzen, die sich in Krankheitsherden selektiv anreichern: Diverse Tumoren entwickeln erhöhte oder erniedrigte Oberflächenkonzentrationen an Rezeptoren z.B. für Wachstumsfaktoren oder Steroidhormone (Sledge, G.W.; Adv. Cancer Res. 38: 61-75 [1983]). Auch bei neurologischen Erkrankungen kommt es zu einer Veränderung der Konzentration von Rezeptoren für Neurotransmitter in bestimmten Bereichen des Hirns (Frost, J.J.; Trends Pharmacol. Sci. 7: 490-496 [1987]). Weiterhin zeigen erkrankte, geschädigte oder zu Tumorzellen transformierte Zellen oft starke Veränderungen ihres Metabolismus und einen Sauerstoffmangel im Innern des Tumors. Die Ausnutzung solcher physiologischer Besonderheiten kann der in vivo-Diagnostik dienen, indem z.B. Hormone, Neurotransmitter oder bestimmte Stoffwechselprodukte wie Fettsäuren, Saccharide, Peptide oder Aminosäuren an Chelatbildner für Tc-99m gekoppelt werden. Auch Substanzen wie Misonidazol (ein Radiosensitizer) bzw. andere in Abwesenheit von Sauerstoff sich zu Radikalen umsetzende Verbindungen können zur spezifischen Anreicherung von radioaktiven Isotopen und somit bildlichen Darstellung von Tumoren oder ischämischen Regionen herangezogen werden. Schließlich ist auch die Kopplung an monoklonale Antikörper möglich, die aufgrund ihrer hohen Spezifität zu einem vielversprechenden Instrument in der Tumordiagnostik geworden sind.

Für die Herstellung von Diagnostika nach dem beschriebenen Prinzip ist es notwendig, daß Chelatbildner für radioaktive Metallionen, insbesondere Tc-99m, an in erkrankten Geweben sich selektiv anreichernde Substanzen gekoppelt werden können.

Da die Isotope des Rheniums (Re-188 und Re-186) ähnliche chemische Eigenschaften wie Tc-99m besitzen, können die Chelatbildner auch zur Komplexierung dieser Isotope verwendet werden. Die genannten Re-Isotope sind ß-Strahler. Somit sind die sich selektiv anreichernden Substanzen mit Rhenium statt mit Technetium komplexiert auch in der Tumortherapie einsetzbar.

Die bisher bekannten Ansätze zur Kopplung von Chelatbildnern an sich selektiv anreichernde Substanzen können vielfach als nicht zufriedenstellend betrachtet werden. Werden die funktionellen Gruppen des Komplexbildners zur Bindung des Chelatbildners an ein solches Molekül benutzt, so kommt es häufig zu einer Abschwächung der Komplexstabilität, d.h. ein diagnostisch nicht tolerierbarer Anteil des Isotops wird aus dem Konjugat freigesetzt. (Brechbiel, M. W. et.al., Inorg. Chem. 25:2772 [1986]). Es ist deswegen notwendig bifunktionelle Komplexbildner herzustellen, d.h. Komplexbildner, die sowohl funktionelle Gruppen zur koordinativen Bindung des gewünschten Metallions als auch eine (andere) funktionelle Gruppe zur Bindung des sich selektiv anreichernden Moleküls tragen. Solche bifunktionellen Liganden ermöglichen eine spezifische, chemisch definierte Bindung von Technetium an verschiedenste biologische Materialien, auch

dann, wenn ein sogenanntes Prelabeling durchgeführt wird. Da nach dieser Methode zuerst die Markierung mit Tc-99m und die Isolierung der Komplexe durchgeführt und erst in einem zweiten Schritt dieser Komplex mit einem sich selektiv anreicherndem Molekül verknüpft wird, erhält man die markierten Verbindungen mit einem hohen Reinheitsgrad.

Es wurden einige Chelatbildner gekoppelt an monoklonale Antikörper (z.B. Europ. Patent App. 0 247 866 und 0 188 256) oder Fettsäuren (Europ. Patent Appl. 0 200 492) beschrieben. Als Chelatbildner werden jedoch die bereits erwähnten $N_2S_2$-Systeme verwendet, die aufgrund ihrer geringen Stabilität wenig geeignet sind. Die etwas stabileren $N_3S$-Chelate zeigten gekoppelt an monoklonale Antikörper keinen so starken Verlust des Tc-99m aus den Konjugaten (J.Lister-James; J.Nucl. Med 30: 793 und Europ. Patent Appl. 0 284 071).

Da sowohl die sich selektiv anreichernden Substanzen in ihren Eigenschaften, sowie auch die Mechanismen, nach denen sie angereichert werden, sehr unterschiedlich sind, ist es weiterhin notwendig, den kopplungsfähigen Chelatbildner variieren und den physiologischen Anforderungen des Kopplungspartners hinsichtlich Lipo- und Hydrophilie, Membranpermeabilität bzw. -impermeabilität etc. anpassen zu können.

Es besteht aus diesen Gründen ein dringlicher Bedarf an stabilen Komplexverbindungen, die gekoppelt oder fähig zur Kopplung an unterschiedliche sich selektiv anreichernde Verbindungen sind.

Aufgabe der Erfindung ist es somit, stabile Chelatbildner, die eine funktionelle Gruppe zur Kopplung an eine sich selektiv anreichernde Verbindung oder eine mit Hilfe dieser funktionellen Gruppe gekoppelte sich selektiv anreichernde Verbindung enthalten, zur Verfügung zu stellen.

Erfindungsgemäß wird diese Aufgabe durch die Verbindungen der allgemeinen Formel I gelöst,

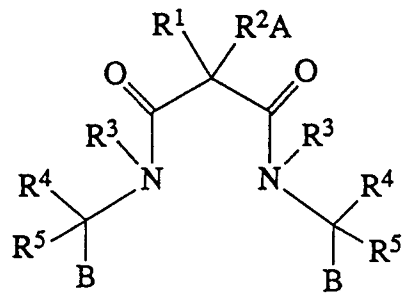

(I)

worin $R^1$ für Wasserstoff oder einen gegebenenfalls mit ein oder zwei Hydroxylgruppen substituierten $C_{1-6}$-Alkylrest,

$R^2$ für einen gegebenenfalls mit einer Hydroxylgruppe substituierten $C_{1-6}$-Alkylenrest,

$R^3$ für ein Wasserstoffatom oder einen $C_{1-6}$-Alkylrest,

$R^4$ für ein Wasserstoffatom, einen gegebenenfalls mit einer Hydroxylgruppe substituierten $C_{1-6}$-Alkylrest bzw. für die unter $R^x$ angegebene Bedeutung steht,

$R^5$ für ein Wasserstoffatom oder einen $C_{1-6}$-Alkylrest steht,

B für einen Pyrrolyl-, einen substituierten Phenylrest der Formel II oder einen substituierten Pyridinrest der Formel III

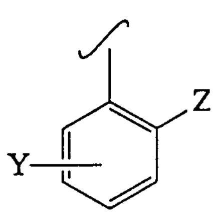

(II)

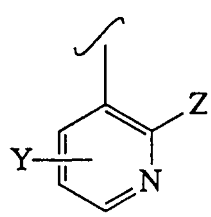

(III)

worin Z eine Hydroxyl-, Amino- oder eine Mercaptogruppe und Y ein Wasserstoffatom, einen Carboxy- oder einen Sulfonylrest bedeuten

oder für einen Nitrosomethylrest der Formel IV

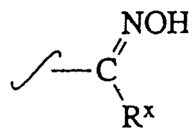

(IV)

steht,

worin $R^x$ einen $C_{1-6}$-Alkylrest, der gegebenenfalls zusammen mit $R^4$ über eine Trimethylen-oder Tetramethylengruppe zu einem 5-bzw. 6-Ring cyclisiert ist, bedeutet

und A für einen Amino-, einen Mercapto-, einen Hydrazino- oder Carbohydrazido-, einen Carboxy-, einen $C_{2-6}$-Alkinyl- oder -Alkenyl-, einen Oxiranyl-, einen fluorierten Phenoxycarbonyl-, einen gegebenenfalls mit einem Natriumsulfatrest substituierten Succinimidoxycarbonyl-, einen Aminophenyl- oder Isothiocyanatophenylrest steht,

wobei die Phenylgruppe gegebenfalls zusätzlich durch einen Carboxy-, einen Chlorsulfonyl- oder einen Sulfonsäurerest substituiert sein kann

oder eine - mit Hilfe der genannten funktionellen Gruppe gegebenenfalls über einen bifunktionellen Linkerrest L gebundene- sich selektiv in Läsionen oder bestimmten Geweben anreichernde Verbindung T enthält

wobei T für monoklonale Antikörper oder deren Fragmente, Hormone, Enzyme, Liganden für Zellmembranrezeptoren, Steroide, Neurotransmitter, Lipide, Saccharide, Aminosäuren und Oligopeptide, Biotin, sowie Radiosensitizer, wie z.B. Misonidazol steht,

sowie deren Komplexe mit- zur Diagnostik und Tumortherapie geeigneten - radioaktiven Metallionen, sowie deren Salze mit anorganischen und organischen Säuren und Basen.

Erfindungsgemäß bevorzugt sind diejenigen Verbindungen gemäß Anspruch 1, in denen $R^4$ und $R^5$ Wasserstoffatome oder Methylreste bedeuten sowie diejenigen Verbindungen gemäß Anspruch 1, in denen A für einen Amino-, einen Mercapto-, einen Carboxy-, einen $C_{2-6}$-Alkinyl- oder -Alkenyl- einen Oxiranyl-, einen fluorierten Phenoxycarbonyl-, einen gegebenenfalls mit einem Natriumsulfatrest substituierten Succinimidoxycarbonyl-, einen Aminophenyl- oder Isothiocyanatophenylrest steht, wobei die Phenylgruppe durch einen weiteren Carboxyl- oder Sulfonsäurerest substituiert sein kann und die gegebenenfalls in A enthaltene sich selektiv anreichernde Verbindung T für monoklonale Antikörper, deren Fragmente, Biotin oder Misonidazol steht.

Überraschenderweise zeigten viele der synthetisierten und mit Tc-99m markierten Chelate eine höhere Stabilität als die vergleichbaren $N_2S_2$-, $N_3S$- und Propylenaminoxim-Chelate. So konnten z.B. bei einer erfindungsgemäßen Substanz (Beispiel 2), die über einen Aminophenylrest an Biotin gekoppelt wurde, keine Zersetzung-Produkte nach 5 Std. beobachtet werden, während dies bei dem vergleichbaren, literaturbekannten HMPAO der Fall war (Hung, J.C. et al.; J. Nucl. Med. 29: 1568, 1988). Auch durch Kompetitionsversuche konnte festgestellt werden, daß die in dieser Erfindung beschriebenen Chelate Tc-99m besser als die vergleichbaren $N_2S_2$-, $N_3S$- und Propylenaminoxim-Chelaten komplexieren. Die in der Erfindung beschriebenen Chelate sind damit eindeutig besser für diagnostische und therapeutische Zwecke geeignet als die bisher bekannten Chelate.

Die Herstellung der Verbindungen gemäß Anspruch 1 erfolgt, indem man

substituierte Malonsäurehalogenide, vorzugsweise Malonsäurechloride der allgemeinen Formel IX,

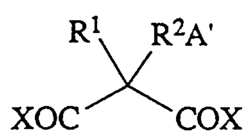

(V)

worin $R^1$, $R^2$ die genannten Bedeutungen haben, X für Halogen und A' für A oder einen in A umwandelbaren Rest steht

mit einem Amin der allgemeinen Formel VI

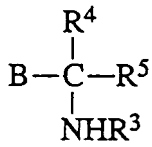 (VI)

in denen $R^3$, $R^4$, $R^5$ und B die oben genannten Bedeutungen haben und in B enthaltene funktionelle Gruppen gegebenenfalls in geschützter Form vorliegen,
in einem aprotischen Lösungsmittel, vorzugsweise Dichlormethan, bei Temperaturen von 0 - 180°C, vorzugsweise bei Raumtemperatur, innerhalb von 2 bis 24 Stunden, vorzugsweise 4 Stunden, gegebenenfalls unter Zusatz von geeigneten Basen, z.B. Triethylamin, umsetzt und vorhandene Schutzgruppen abspaltet
und in den so erhaltenen Verbindungen die Gruppe A' gegebenenfalls zu A umwandelt - gegebenenfalls nach Schutz der freien Aminogruppen und funktionellen Gruppen Z mit Schutzionen, z.B. als Cu-Komplex - generiert und anschließend gewünschtenfalls die so erhaltenen Verbindungen über diese funktionelle Gruppe an sich selektiv anreichernde Verbindungen T koppelt und den Substituenten B gewünschtenfalls mit dem jeweils gewünschten radioaktiven Isotop komplexiert, wobei vorher im Produkt gegebenenfalls vorhandene Schutzionen nach an sich literaturbekannten Methoden entfernt werden und die Reihenfolge der Schritte Komplexierung mit radioaktiven Isotopen und Kopplung an T vertauscht werden kann.

Als Hydroxyschutzgruppen kommen z.B. die Benzyl-, 4-Methoxybenzyl-, 4-Nitrobenzyl-, Trityl-, Diphenylmethyl-, Trimethylsilyl-, Dimethyl-t-butylsilyl- und Diphenyl-t-butylsilylgruppe infrage. Im Falle von Polyolen können die Hydroxygruppen auch in Form von Ketalen mit z.B. Aceton, Acetaldehyd, Cyclohexanon oder Benzaldehyd geschützt sein. Ferner können die Hydroxygruppen auch z.B. als THP-Ether, $\alpha$-Alkoxyethylether, MEM-Ether oder als Ester mit aromatischen oder aliphatischen Carbonsäuren, wie z.B. Essigsäure oder Benzoesäure, vorliegen. Die Hydroxyschutzgruppen können nach den dem Fachmann bekannten Literaturmethoden, z.B. durch Hydrogenolyse, reduktive Spaltung mit Lithium/Ammoniak, Säurebehandlung der Ether und Ketale oder Alkalibehandlung der Ester freigesetzt werden (siehe z.B. "Protective Groups in Organic Synthesis", T.W. Greene, John Wiley and Sons 1981).

Als Aminoschutzgruppen kommen z.B. Trifluoracetyl-, t-Butoxycarbonyl-, 2,2,2-Trichloroethoxycarbonyl-, Benzoxycarbonyl- und Acetylgruppen infrage. Die Aminoschutzgruppen können nach literaturbekannten Methoden, z.B. durch basische oder saure Hydrolyse, reduktive Spaltung mit Zink in Essigsäure oder Hydrogenolyse abgespalten werden.

Als Mercaptoschutzgruppen kommen $C_{1-6}$-Alkylreste oder Benzylthioether infrage. Die Abspaltung der Mercaptoschutzgruppen erfolgt wahlweise mit Alkalialkylthiolaten, Alkalialkoholaten oder Alkalimetallen, vorzugsweise mit Natriummethylthiolat in einem polaren Lösungsmittel, vorzugsweise in HMPT, DMF oder Dimethylacetamid oder durch reduktive Spaltung mit Natrium/Ammoniak.
Die funktionelle Gruppe im Rest A ist dazu geeignet, eine stabile Verbindung zu Proteinen oder anderen sich selektiv anreichernden Molekülen herzustellen. Durch die entsprechende Wahl der funktionellen Gruppe im Rest A gelingt die Kopplung unter schonenden Reaktionsbedingungen, die die biologische Funktion und/oder Selektivität nicht beeinflussen. Die Kopplung an die gewünschten Verbindungen erfolgt ebenfalls nach an sich bekannten Methoden, (z.B. Fritzberg et al.; J. Nucl. Med.: 26, 7 [1987]), beispielsweise durch Reaktion der entsprechenden funktionellen Gruppe im Rest A mit nucleophilen Gruppen des sich selektiv anreichernden Moleküls oder, wenn es sich bei der entsprechenden funktionellen Gruppe im Rest A selbstum ein Nucleophil handelt, mit aktivierten Gruppen des sich selektiv anreichernden Moleküls.

Die Kopplung an Steroide mit einer 17-Jodvinylfunktion kann über eine Ethinylfunktion des Komplexbildners erfolgen. Dabei ist es unerheblich, ob die E- oder Z-Verbindung eingesetzt wird. Die Entfernung der gegebenenfalls vorhandenen Schutzgruppen kann sowohl vor als auch nach der Kopplung erfolgen. Erfindungsgemäß bevorzugt werden 17$\alpha$-Ethinylestradiol sowie 17$\alpha$-Ethinylnortestosteron. Diese Verbindungen haben Bedeutung für die Diagnose steroidabhängiger Tumore erlangt [J.K.Mazaitis, B.E.Francis, W.C.Eckelmann, R.E.Gibson, R.C.Reba, J.W.Barnes, G.E.Bentley, P.M.Grant, H.A.O'Brien jr.; J. Labelled Compd. Radiopharm. 1980, 18, 1033].
Die Darstellung von 17 -Jodvinylsteroiden kann nach literaturbekannten Verfahren erfolgen [z.B. H. Hofmeister, H. Laurent, P.-E. Schulze und R. Wiechert, Tetrahedron 42, 3575 (1986)].

Die funktionelle Gruppe im Rest A repräsentiert jeden Substituenten, der einerseits eine Kopplung an ein sich selektiv anreicherndes Molekül unter milden Bedingungen erlaubt (z.B. durch Acylierung oder Amidierung) sowie jede aktivierte Gruppe, die mit nucleophilen Gruppen von Proteinen, Antikörpern, Hormonen oder anderen Biomolekülen reagieren kann, wie der Amino-, Phenol-, Sulfhydryl-, Formyl- oder

Imidazol-Gruppe. Unter aktivierter Gruppe wird eine Funktion verstanden, die fähig ist, unter Bildung eines Konjugates mit einem nucleophilen Substituenten eines sich selektiv anreichernden Moleküls oder des Komplexliganden selbst in wässriger Lösung innerhalb einer angemessen kurzen Zeit, unter Reaktionsbedingungen, die weder Denaturierung noch Verlust der biologischen Aktivität zufolge haben, zu reagieren. Beispiele dafür sind Imidester, Alkylimidester, Amidoalkylimidester, Succinimidester, Acylsuccinimide, Phenolester, substituierte Phenolester, Tetrafluorphenolester, Anhydride, und Michael-Akzeptoren. Die funktionelle Gruppe im Rest A ist bevorzugt ein aktivierter Ester (wie Phenol- oder Imid-Ester), ein Mercaptan oder Isothiocyanat, insbesondere für die Kopplung mit nucleophilen Gruppen von Aminosäuren oder ein aliphatisches oder aromatisches primäres Amin für die Kopplung an Kohlenhydratresten von Proteinen.

Handelt es sich bei der funktionellen Gruppe im Rest A selbst um ein Nucleophil, kann sie mit aktivierten Gruppen eines sich selektiv anreichernden Moleküls reagieren, wobei auch mit sogenannten "Cross-linking-reagents" umgesetzte Gruppen des Moleküls mit eingeschlossen sind, wie homobifunktionelle Imidoester, homobifunktionelle N-Hydroxysuccinimidester (NHS) und heterobifunktionelle "Cross-Linker", die unterschiedliche funktionelle Gruppen, wie NHS-Ester, Pyridyl-Disulfide und aktivierte Halogene, wie$\alpha$-Keto-Halogenide enthalten. Solche Cross-Linker sind kommerziell erhältlich.

Als Kopplungspartner (= Verbindung T) sind u.a. verschiedene Biomoleküle vorgesehen: Liganden, die an spezifische Rezeptoren binden und so ein in ihrer Rezeptordichte verändertes Gewebe erkennenkönnen; hierzu gehören u.a. Peptid- und Steroidhormone und Neurotransmitter. Mit Liganden für Steroidhormon-Rezeptoren wurde die Möglichkeit einer verbesserten Diagnostik von Brust- und Prostatacarcinomen aufgezeigt (S.J. Brandes & J.A. Katzenellenbogen, Nucl. Med. Biol. 15: 53, 1988). Vielfach konnten mit Positronen-emittierenden Isotopen markierte Liganden für Neurorezeptoren zur Diagnostik verschiedener Hirnerkrankungen herangezogen werden (J.J. Forst, Trends in Pharmacol. Sci. 7: 490, 1987). Weitere Biomoleküle sind in den Metabolismus der Zellen einschleusbare Metaboliten, die einen veränderten Stoffwechselerkennbarmachen; hierzu gehören beispielsweise Fettsäuren, Saccharide, Peptide und Aminosäuren. Fettsäuren gekoppelt an die unstabileren $N_2S_2$-Chelatbildner wurden in der EPA 0 200 492 beschrieben. Andere Stoffwechselprodukte wie Saccharide (Dexoxyglucose), Lactat, Pyruvat und Aminosäuren (Leucin, Methylmethionin, Glycin) wurden mit Hilfe der PET-Technik zur bildlichen Darstellung von veränderten Stoffwechselvorgängen herangezogen (R. Weinreich, Swiss Med. 8, 10, 1986). Auch nichtbiologische Substanzen wie Misonidazol und seine Derivate, die sich in Geweben bzw. Gewebeteilen mit reduzierter Sauerstoffkonzentration irreversibel an Zellbestandteile binden, können zur spezifischen Anreicherung von radioaktiven Isotopen und somit bildlichen Darstellung von Tumoren oder ischämischen Regionen herangezogen werden (M.E. Shelton, J. Nucl. Med. 30: 351, 1989). Schließlich ist auch die Kopplung der bifunktionellen Chelatbildner an monoklonale Antikörper bzw. deren Fragmente möglich. Die Biotin enthaltenden erfindungsgemäßen Verbindungen ermöglichen die Bindung der radioaktiven Konjugate an Avidin- oder Streptavidin-haltige Substanzen. Dies kann genutzt werden, um Antikörper-Streptavidin-Konjugate am Tumor anzureichern und die radioaktive Biotin-haltige Komponente erst später zu applizieren, was zu einer verringerten Exposition des Patienten mit radioaktiver Strahlung führt (D.J. Hnatowich et al., J. Nucl. Med. 28: 1294, 1987). Durch Komplexierung der Konjugate mit Tc-99m bzw. Rhenium-Isotopen kann eine Diagnostik und Therapie von Tumoren ermöglicht werden.

Es ist unerheblich, ob eine Markierung der Chelatbildner mit Tc-99m oder einem Rhenium-Isotop vor oder nach der Kopplung an das sich selektiv anreichernde Molekül durchgeführt wird. Für eine Kopplung an das sich selektiv anreichernde Molekül nach einer Komplexierung ist jedoch Voraussetzung, daß die Umsetzung des radioaktiven Komplexes mit der sich anreichernden Verbindung schnell, unter schonenden Bedingungen und nahezu quantitativ abläuft, sowie keine anschließenden Aufreinigung erforderlich ist.

Die Herstellung der erfindungsgemäßen pharmazeutischen Mittel erfolgt in an sich bekannter Weise, in dem man die erfindungsgemäßen Komplexbildner unter Zusatz eines Reduktionsmittels, vorzugsweise Zinn-(II)-salzen wie -chlorid oder -tartrat - und gegebenenfalls unter Zugabe der in der Galenik üblichen Zusätze -in wäßrigem Medium löst und anschließend sterilfiltriert.

Geeignete Zusätze sind beispielsweise physiologisch unbedenkliche Puffer (z.B. Tromethamin), geringe Zusätze von Elektrolyten (z.B. Natriumchlorid), Stabilisatoren (z.B. Gluconat, Phosphate oder Phosphonate). Das erfindungsgemäße pharmazeutische Mittel liegt in Form einer Lösung oder in lyophilisierter Form vor und wird kurz vor der Applikation mit einer Lösung Tc-99m-Pertechnetat, eluiert aus kommerziell erhältlichen Generatoren, oder einer Perrhenatlösung versetzt.

Bei der nuklearmedizinischen In-vivo-Anwendung werden die erfindungsgemäßen Mittel in Mengen von $1 \cdot 10^{-5}$ bis $5 \cdot 10^{4}$ nmol/kg Körpergewicht, vorzugsweise in Mengen zwischen $1 \cdot 10^{-3}$ und $5 \cdot 10^{2}$ nmol/kg Körpergewicht dosiert. Ausgehend von einem mittleren Körpergewicht von 70 kg beträgt die Radioaktivitätsmenge für diagnostische Anwendungen zwischen 0,05 und 50 mCi, vorzugsweise 5 bis 30 mCi pro Applikation. Für therapeutische Anwendungen werden zwischen 5 und 500 mCi, vorzugsweise 10 - 350 mCi

appliziert. Die Applikation wird normalerweise durch intravenöse, intraarterielle, peritoneale oder intratumorale Injektion von 0,1 bis 2 ml einer Lösung der erfindungsgemäßen Mittel erfolgen. Bevorzugt ist die intravenöse Applikation.

Die nachfolgenden Beispiele dienen der näheren Erläuterung des Erfindungsgegenstandes.

**Beispiel 1:**

2-Methyl-2-(4-nitrobenzyl)-malonsäuredimethylester [1]

Es werden 11.5 g (0.5 mol) Natrium untereinem Stickstoffstrom in einen trockenen 1000 ml fassenden Dreihalskolben mit Rückflußkühler und Trockenrohr sowie Tropftrichter mit Druckausgleich gegeben. Anschließend werden vorsichtig 350 ml Methanol zugetropft und solange gerührt, bis das Natrium vollständig gelöst ist. In die noch warme Natriummethanolat-Lösung wird sehr langsam die methanolische Lösung von 87 g(0.5 mol) Methylmalonsäurediethylester zugetropft. Nach beendeter Zugabe wird noch 30 Minuten gerührt und dann portionsweise mit Hilfe eines Pulvertrichters das 4-Nitrobenzylbromid zugegeben. Nachdem sich alles gelöst hat wird zunächst 2 Stunden unter Rückfluß erhitzt und anschließend über Nacht bei Raumtemperatur gerührt. Das Lösungsmittel wird am Rotationsverdampfer abgezogen, der Rückstand mit Wasser versetzt und mehrmals mit Ethylacetat (je 250ml) extrahiert. Die vereinigten organischen Extrakte werden mit gesättigter Kochsalzlösung gewaschen und über Natriumsulfat getrocknet. Nach Abziehen des Lösungsmittels verbleiben schwach gelbe Kristalle. Die Umkristallisation erfolgt aus Dimethylformamid (DMF)/Wasser.

Schmelzpunkt: 94.5 - 95.0 ° C
Ausbeute: 74%
$^1$H-NMR-Daten in DMSO/TMS
1,4 ppm (s,3H,Me); 3,5 ppm (s,2H,$CH_2$Ar); 3,8 ppm (s,6H,MeOOC); 7,2 ppm (d,2H, ArH); 8,2 ppm (d, 2H,ArH)

2-Methyl-2-(4'-nitrobenzyl)-malonsäure [2]

Es werden 28,1 g 2-Methyl-2-(4'nitrobenzyl)malonsäureethylester (0,1 mol) in 100 ml Methanol gelöst und dazu eine Lösung von 20 g NaOH (0,5 mol) in 50 ml Wasser langsam zugetropft. Anschließend wird 2 Stunden im Wasserbad auf 50 ° C erwärmt, das Lösungsmittel am Rotationsverdampfer abgezogen und der Rückstand in Wasser aufgenommen. Es wird mit halbkonzentrierter Salzsäure angesäuert und die freie Carbonsäure mit Chloroform extrahiert, gewaschen und getrocknet.
Ausbeute: 98%
$^1$H-NMR-Daten in DMSO/TMS
1,4 ppm (s,3H,Me); 3,1 ppm (d,2H,$CH_2$Ar); 7,4 ppm (d,2H,ArH); 8,2 ppm (d,2H,ArH).

2-Methyl-2-(4'-nitrobenzyl)malonsäuredichlorid [3]

Die Mischung aus 25,3 g [2] und 35,7 g Thionylchlorid und einem Tropfen DMF wird unter Feuchtigkeitsausschluß unter Rühren zum Sieden erhitzt. Nach Beendigung der Gasentwicklung wird der Überschuß Thionylchlorid bei Raumtemperatur im Vakuum abgezogen und der Rückstand sofort weiter umgesetzt.
Ausbeute: 91%

N,N'-Bis(2-methoxybenzyl)-2-methyl-2-(4'-nitrobenzyl)malonsäurediamid [4]

Zu der Lösung aus 5,5 g 2-Methoxybenzylamin (40mmol) und 4,3 g Triethylamin (42mmol) in 100 ml Methylenchlorid wird unter Ausschluß von Feuchtigkeit die Lösung von 7,1 g [3] (24mmol) in 100 ml Methylenchlorid vorsichtig zugetropft. Anschließend wird sechs Stunden bei Raumtemperatur gerührt und die Reaktionsmischung mehrmals mit Wasser gewaschen, getrocknet, eingeengt und aus 2-Propanol umkristallisiert
Ausbeute: 78%
$^1$H-NMR-Daten in $CDCl_3$/TMS
1,3 ppm (s,3H,$CH_3$); 3,2 ppm (s,2H,$CH_2$Ar); 3,8 ppm (s,6H,$CH_3$OAr); 4,4 ppm (d,4H,Ar$CH_2$NH); 6,8-7,4 ppm (m,10H,ArH); 7,8 ppm (d,2H, ArH)

N,N'-Bis(2-hydroxybenzyl)-2-methyl-2-(4'-nitrobenzyl)malonsäurediamid [5]

Zu einer Lösung von 4,90 mg [4] (10mmol) in 150 ml wasserfreiem Methylenchlorid werden bei 0 ° C unter einer Argon-Atmosphäre 17 g Bortribromid (70 mmol) in 200 ml Methylenchlorid langsam zugetropft und anschließend auf 40 ° C erwärmt. Nach 2 Stunden wird mit Wasser hydrolisiert, mit Kaliumcarbonatlösung alkalisiert und mit Methylenchlorid extrahiert. Nach Trocknen und Abziehen des Lösungsmittels verbleibt ein schwach gelber Rückstand, der aus 2-Propanol umkristallisiert wird.
Ausbeute: 80%
$^1$H-NMR-Daten in $CDCl_3$/TMS
1,3 ppm (s,3H,$CH_3$); 3,0 ppm (s,2H,$CH_2$Ar); 4,4 ppm (d,4H,Ar$CH_2$NH); 6,3 ppm (d,2H,ArH); 6,6 ppm (d,2H,ArH); 6,8 ppm-7,3 ppm (m,6H,ArH); 7,8 ppm (d,2H,ArH); 9,0 ppm (s,2H,NH)

N,N'-Bis(2-hydroxybenzyl)-2-(4'-aminobenzyl)-2-methylmalonsäurediamid [6]

In einem 250 ml Zweihalskolben werden 230 mg 10% Pd/C in 80 ml MeOH suspendiert und mit Wasserstoff gesättigt. Anschließend wird die Lösung von 1,15 g [5] (2.5 mmol) in 4 ml Methanol mit einer 5 ml Einwegspritze durch ein Septum zugegeben. Nach Beendigung der Wasserstoffaufnähme wird vom Katalysator abgetrennt und das Lösungsmittel im Vakuum abgezogen. Es verbleiben weiße Kristalle. Die Umkristallisation erfolgt aus Methanol.
Ausbeute: 94%
$^1$H-NMR-Daten in $CDCl_3$/TMS
1,3 ppm (s,3H,$CH_3$); 3,0 ppm (s,2H,$CH_2$Ar); 4,4 ppm (d,4H,Ar$CH_2$NH); 6,3 ppm (d,2H,ArH); 6,6 ppm (d,2H,ArH); 6,8 ppm-7,3 ppm (m,8H,ArH); 9,0 ppm (s,2H,NH)

N,N'-Bis(2-hydroxybenzyl)-2-(4'-isothiocyanatobenzyl)-2-methylmalonsäurediamid [7]

Eine 85%ige Lösung von Thiophosgen in Tetrachlorkohlenstoff (0,2ml, 2,23 mmol) wird zu der Lösung von 257 mg [6] (0,5 mmol) in 4 ml Salzsäure (3 M) gegeben. Das Reaktionsgemisch wird 3 Stunden bei Raumtemperatur gerührt und dann im Vakuum bis zur Trockene eingeengt.
Ausbeute: 86%
$^1$H-NMR-Daten in $CDCl_3$/TMS
1,3 ppm (s,3H,$CH_3$); 3,0 ppm (s,2H,$CH_2$Ar); 4,3 ppm (d,4H,Ar$CH_2$NH); 6,7 ppm-7,3 ppm (m,12H,ArH); 7,7 ppm (t,2H,NH); 8,8 ppm (s,2H,OH)

**Beispiel 1a**

N,N'-Bis(2-hydroxybenzyl)-2-[4'-(Biotincarbamoyl)benzyl]-2-methylmalonsäurediamid [8]

Zu einer Lösung von 514 mg [6] (1 mmol) in 10 ml DMSO werden 680 mg NHS-Biotin (2 mmol) unter Rühren dazugegeben und 3 Stunden auf 50 ° C erwärmt. Nach Abkühlen auf Raumtemperatur wird noch 15 Stunden gerührt. Anschließend wird das Lösungsmittel im Vakuum abgezogen und der Rückstand mittels MPLC gereinigt (Kieselgel, Dichlormethan/Ethanol/konzentriertes Ammoniak 10:20:1)
Ausbeute: 59%
$^1$H-NMR-Daten in DMSO/TMS
1,3 ppm (s,3H,$CH_3$); 1,3-1,7 pm (m,6H,$CH_2$); 2,3 ppm (t,2H,$CH_2$CO); 2,8 ppm (m,2H, $CH_2$S); 3,0 ppm (s,2H,$CH_2$Ar); 3,1 ppm (m,1H, CHS); 4,1 ppm (m,1H,CHNHCO); 4,3 ppm (m,5H,Ar$CH_2$NH + CHNHCO); 6,7 ppm-7,3 ppm (m,12H,ArH).

**Beispiel 1b**

N,N'-Bis(2-hydroxybenzyl)-2-[4'-{{4-(N-maleimidomethyl)cyclohexan}carbamoyl}benzyl]-2-methylmalonsäurediamid [9]

Diese Verbindung wird entsprechend der Verbindung [8] dargestellt.
Ausbeute: 45%
$^1$H-NMR-Daten in DMSO/TMS
1,3 ppm (s,3H,$CH_3$); 1,1-1,9 ppm (m,9H,$CH_2$); 2,5 ppm (m,1H, CHCO); 3,0 ppm (s,2H,$CH_2$Ar); 3,3 ppm (CH$CH_2$N); 4,4 ppm (d,4H,Ar$CH_2$NH); 6,3 ppm (d,2H,ArH); 6,6 ppm (d,2H,ArH); 6,8 ppm-7,3 ppm (m,10H,ArH + CH = CH).

**Beispiel 1c**

Tc-99m-Komplex von [6]

Eine Lösung von 5,0 mg [6] in 5 ml Wasser wird mit EtOH auf 10 ml aufgefüllt. 400 µl dieser Lösung werden mit 80 µl einer gesättigten Zinntartrat-Lösung und 8 mCi Tc-99m-Pertechnetat aus einem Mo99/Tc99m-Generator versetzt und 5 Minuten stehen gelassen. Anschließend wird diese Lösung je dreimal mit 2 ml Chloroform extrahiert, die organische Phase über eine kurze Natriumsulfatsäule getrocknet, mit 50 µl Thiophosgen versetzt und 5 Minuten bei Raumtemperatur inkubiert. Anschließend wird mit 100 µl Isopropanol versetzt, mit Argon das Chloroform abgedampft und zu dem Rückstand 900 µl einer 1% igen PVP-Lösung in Wasser gegeben.

Kopplung des Isothiocyanat-haltigen Tc-99m-Komplexes an Proteine

Dergereinigte Antikörper (10 mg/ml) wird bei pH 3,5 für 2 Std. mit 25 µg/ml Pepsin behandelt und anschließend mittels FPLC isoliert. Vorder Kopplung mit dem Chelatbildner werden die Fragmente bei 4°C für 12 - 24 Std. gegen 0,1 M $KH_2PO_4$/0,1 M $NaHCO_3$, pH 8,5, dialysiert. Die Proteinkonzentration wird auf 10 mg/ml eingestellt Der hergestellte Tc-99m-Komplex wird in einem molaren Verhältnis 1:10 (Komplex : Protein) zur Proteinlösuung hinzugegeben. Zur Konjugatbildung wird die Mischung für eine Stunde bei 37°C inkubiert.

**Beispiel 2**

N,N'-Bis[(5-carboxy-2-methoxy)benzyl)]-2-methyl-2-(4'-nitrobenzyl)-malonsäurediamid [10]

Diese Verbindung wird entsprechend der Verbindung [4] dargestellt
Ausbeute: 56%
$^1$H-NMR-Daten in DMSO/TMS
1,3 ppm (s,3H,$CH_3$); 3,2 ppm (s,2H,$CH_2$Ar); 3,8 ppm (s,6H,$CH_3$OAr); 4,5 ppm (d,4H,Ar$CH_2$NH); 6,9-8,0 ppm (m,8H,ArH); 8,2 ppm (d,2H, ArH)

N,N'-Bis[5'-carboxy-2'-hydroxy)benzyl]-2-(4'-nitrobenzyl)-2-methylmalonsäurediamid][11]

Diese Verbindung wird entsprechend der Verbindung [5] dargestellt.
Ausbeute: 79%
$^1$H-NMR-Daten in DMSO/TMS
1,3 ppm (s,3H,$CH_3$); 3,1 ppm (s,2H,$CH_2$Ar); 4,4 ppm (d,4H,Ar$CH_2$NH); 6,8 - 8,0 ppm (m,10H,ArH).

N,N'-Bis[(5'carboxy-2'-hydroxy)benzyl]-2-(4'-aminobenzyl)-2-methylmalonsäurediamid[12]

Diese Verbindung wird entsprechend der Verbindung [6] dargestellt.
Ausbeute: 92%
$^1$H-NMR-Daten in DMSO/TMS
1,3 ppm (s,3H,$CH_3$); 3,0 ppm (s,2H,$CH_2$Ar); 4,4 ppm (d,4H,Ar$CH_2$NH); 6,8 - 7,9 ppm (m,10H,ArH).

N,N'-Bis[(5'-carboxy-2'-hydroxy)benzyl]-2-(4'-isothiocyanatobenzyl)-2-methylmalonsäurediamid[13]

Diese Verbindung wird entsprechend der Verbindung [7] dargestellt.
Ausbeute: 83%
$^1$H-NMR-Daten in DMSO/TMS
1,3 ppm (s,3H,$CH_3$); 3,0 ppm (s,2H,$CH_2$Ar); 4,4 ppm (d,4H,Ar$CH_2$NH); 6,8 - 7,9 ppm (m,10H,ArH).

**Beispiel 3**

2-[4'-(Tetrahydro-2-pyranyloxy)butyl]-malonsäuredimethylester [14]

7,9 g (0.3 mol) Natrium werden in einem 500 ml Dreihalskolben mit Rückflußkühler und Trockenrohr sowie Tropftrichter mit Druckausgleich unter einem Stickstoffstrom gegeben. Anschließend werden vorsichtig 250 ml Methanol wasserfrei zugetropft und solange gerührt, bis das Natrium vollständig gelöst ist. In die noch warme Natriummethanolat-Lösung wird sehr langsam die methanolische Lösung von 95.4g (0.58 mol) Malonsäurediethylesters zugetropft. Nach beendeter Zugabe wird noch 1 Stunde unter Rückfluß gekocht und dann langsam 0.5 mol 1-Chlor-4-tetrahydro-pyranyloxybutan zugetropft. Nach beendeter Zugabe wird über Nacht unter Rückfluß gekocht. Das Lösungsmittel wird am Rotationsverdampfer abgezogen, der Rückstand mit Wasser versetzt und mehrmals mit Ethylacetat extrahiert. Die vereinigten organischen Extrakte werden mit gesättigter Kochsalzlösung gewaschen und über Natriumsulfat getrocknet. Nach Abziehen des Lösungsmittels verbleibt ein farbloses Öl. Nach Destillation im Vakuum verbleiben 66.4 g des substituierten Malonesters.
Ausbeute: 70%
$^1$H-NMR-Daten in $CDCl_3$/TMS
1,6-2 ppm (m,12H,$CH_2$); 3,5-3,7 ppm (m,5H,CH + OTHP); 3,7 ppm (s,6H,COOMe); 4,6 ppm (t,1H,OTHP)

2-[4'-(Tetrahydro-2-pyranyloxy)butyl]-malonsäure [15]

Diese Verbindung wird entsprechend der Verbindung [2] dargestellt.
Ausbeute: 93%
$^1$H-NMR-Daten in DMSO/TMS
1,3-2,1 ppm (m,12H,$CH_2$); 3,1-4,0 ppm (m,5H,CH,OTHP); 4,5 ppm (t, 1H,OTHP);

2-[4'-(Tetrahydro-2-pyranyloxy)butyl]-malonsäurechlorid [16]

Diese Verbindung wird entsprechend der Verbindung [3] dargestellt.
Ausbeute: 89%

N,N'-Bis[2-(nitrobenzyl)]-2-[4'-(tetrahydro-2-pyranyloxy)butyl]malonsäurediamid [17]

Zu einer Lösung von 6,3 g [16] (12mmol) und 2,5 g Triethylamin in 100 ml Dichlormethan werden tropfenweise 3,5 g 2-Nitrobenzylamin (23mmol) in 150 ml Dichlormethan innerhalb einer Stunde langsam zugetropft. Die Lösung wird anschließend auf 0 °C gekühlt, mit Wasser versetzt und die wässrige Phase mehrmals mit Dichlormethan extrahiert und über Natriumsulfat getrocknet. Nach abziehen des Lösungsmittels verbleibt ein gelbes Öl.
Ausbeute: 68%
$^1$H-NMR-Daten in $CDCl_3$/TMS
1,6-2 ppm (m,12H,$CH_2$); 3,5-3,7 ppm (m,5H,CH + OTHP); 4,5 ppm (s,4H, $NHCH_2Ar$); 4.6 ppm (t,1H,OTHP); 7,7-7,9 ppm (m,6H,ArH); 8,3 ppm (d, 2H,ArH)

N,N'-Bis[2-(nitrobenzyl)]-2-(4-hydroxybutyl)malonsäurediamid [18]

Eine Lösung von 26 g [17] (50 mmol) in 50 ml Methanol wird zu einer Lösung von 8 ml Schwefelsäure in 150 ml Methanol getropft und 15 Stunden bei Raumtemperatur gerührt. Es wird auf 0 ° C abgekühlt, neutralisiert, mit Dichlormethan extrahiert, getrocknet und eingeengt.
Ausbeute: 69%
$^1$H-NMR-Daten in $CDCl_3$/TMS
1,4-1,6 ppm (m,6H,$CH_2$); 3,5 ppm (t,1H,CH); 3,6 ppm (t,2H,$CH_2OH$); 4,5 ppm (s,4H,$NCH_2Ar$); 7,7-7,9 ppm (m,6H,ArH); 8,3 ppm (d,2H,ArH)

N,N'-Bis[2-(nitrobenzyl)]-2-(4-methansulfonylbutyl)malonsäurediamid [19]

Zu einer Lösung von 1,8 g [18] (4mmol) und 606 mg Triethylamin (6mmol) in 25 ml Dichlormethan wird bei 0 °C 500 mg Methansulfonylchlorid gegeben. Nach 2 Stunden wird die Lösung mit Eiswasser versetzt und mehrmals mit Dichlormethan extrahiert, gewaschen und getrocknet Nach dem Einengen verbleibt ein gelbes Öl.
Ausbeute: 90%
$^1$H-NMR-Daten in $CDCl_3$/TMS
1,4-1,6 ppm (m,6H,$CH_2$); 3,0 ppm (s,3H,Me); 3,5 ppm (t,1H,CH); 4,0 ppm (t,2H,$CH_2OR$); 4,5 ppm (s,4H,$NCH_2Ar$); 7,7-7,9 ppm (m,6H,ArH); 8,3 ppm (d,2H,ArH)

N,N'-Bis[2-(nitrobenzyl)]-2-(4-thiocyanatobutyl)malonsäurediamid [20]

Zu einer Lösung von 22,5 g [19] (43 mmol) in 100 ml Methanol wird ein Überschuß an Kaliumthiocyanat (100mmol) gegeben und 36 Stunden unter Rückfluß erhitzt. Anschließend wird zur Trockene eingeengt und der Rückstand in Chloroform aufgenommen und über eine Flash-Chromatographie (Chloroform) gereinigt
Ausbeute: 64%
$^1$H-NMR-Daten in $CDCl_3$/TMS
1,4-1,6 ppm (m,6H,$CH_2$); 3,1 ppm (t,2H,$CH_2$SCN); 3,5 ppm (t,1H,CH); 4,5 ppm (s,4H,$NCH_2$Ar); 7,7-7,9 ppm (m,6H,ArH); 8,3 ppm (d,2H,ArH)

N,N'-Bis[2-(aminobenzyl)]-2-(4-thiocyanatobutyl)malonsäurediamid [21]

Diese Verbindung wird entsprechend der Verbindung [6] dargestellt.
Ausbeute: 78%
$^1$H-NMR-Daten in DMSO/TMS
1,4-1,6 ppm (m,6H,$CH_2$); 3,1 ppm (t,2H,$CH_2$SCN); 3,5 ppm (t,1H,CH); 4,3 ppm (s,4H,$NCH_2$Ar); 6,6-7,6 ppm (m,8H,ArH)

N,N'-Bis[2-(aminobenzyl)]-2-(4-mercaptobutyl)malonsäurediamid [22]

Eine Lösung von 13 g [20] (28mmol) in Methanol wird langsam zu einer Lösung von 2,7 g Natriumborhydrid (70mmol) in 50 ml Wasser getropft. Nach beendeter Zugabe wird noch 2 Stunden bei 50 ° C gerührt, abgekühlt, mit 2N Salzsäure neutralisiert und mit Dichlormethan mehrmals extrahiert. Nach dem Trocknen über Natriumsulfat und Einengen verbleibt ein gelbes Öl.
Ausbeute: 41%
$^1$H-NMR-Daten in $CDCl_3$/TMS
1,4-1,6 ppm (m,6H,$CH_2$); 2,4 ppm (t,2H,$CH_2$SH); 3,4 ppm (t,1H,CH); 4,5 ppm (s,4H,$NCH_2$Ar); 6,6-7,6 ppm (m,8H,ArH)

**Beispiel 4:**

2-(4-Nitrobenzyl)-malonsäurediethylester [23]

Es werden 21.4 g Lithiumdiisopropylamid zu 210 ml wasserfreiem Tetrahydrofuran unter einem Stickstoffstrom in einem trockenen Dreihalskolben mit Trockenrohr und Tropftrichter mit Druckausgleich gegeben. Anschließend werden bei Raumtemperatur 58.0 g Malonsäurediethylester in 100 ml wasserfreiem Tetrahydrofuran innerhalb von 40 Minuten zugetropft. Nach 30 Minuten wird die Reaktionslösung auf -62 ° C abgekühlt und 39.1 g 4-Nitrobenzylbromid in Tetrahydrofuran langsam unter starkem Rühren zugetropft, eine weitere Stunde bei - 62 ° C gerührt und anschließend wird der gebildete Niederschlag in der Kälte abfiltriert. Das Filtrat wird am Rotationsverdampfer eingeengt, der Rückstand in 300 ml Ethanol bei 65 ° C in Lösung gebracht und vom unlöslichen Rückstand abgetrennt. Nach dem Abkühlen erhält man 34.7 g schwach gelbliche Kristalle.
Ausbeute: 65%
$^1$H-NMR-Daten in $CDCl_3$/TMS
1,2 ppm (t,6H,$CH_2CH_3$); 3,3 ppm (d,2H,$CH_2$Ar); 3,6 ppm (t,1H,CH); 4,1 ppm (q,4H,$CH_2CH_3$); 7,4 ppm (d,2H,ArH); 8,1 ppm (d,2H, ArH)

2-(4-Nitrobenzyl)-malonsäure [24]

Diese Verbindung wird entsprechend der Verbindung [2] dargestellt.
Ausbeute 98%
$^1$H-NMR-Daten in DMSO/TMS
3,1 ppm (d,2H,$CH_2$Ar); 3,4 ppm (t,1H,CH); 7.1 ppm (s,4H,$NH_2$); 7,4 ppm (d,2H,ArH); 8,2 ppm (d,2H,ArH)

2-(4-Nitrobenzyl)-malonsäuredichlorid [25]

Diese Verbindung wird entsprechend der Verbindung [3] dargestellt.
Ausbeute: 76%

N,N'-Bis[Methyl-2-pyrrolyl]-2-(4'-nitrobenzyl)malonsäurediamid[26]

Zu der Lösung aus 3,8 g 2-Aminomethylpyrrol (40mmol) und 4,3 g Triethylamin (42mmol) in 100 ml Methylenchlorid wird unter Ausschluß von Feuchtigkeit die Lösung von 6,6 g [25] (24mmol) in 100 ml Methylenchlorid vorsichtig zugetropft. Anschließend wird sechs Stunden bei Raumtemperatur gerührt und die Reaktionsmischung mehrmals mit Wasser gewaschen, getrocknet, eingeengt und aus 2-Propanol umkristallisiert
Ausbeute: 76%
$^1$H-NMR-Daten in DMSO/TMS
3,1 ppm (d,2H,$CH_2$Ar); 3,4 ppm (t,1H,CH); 3,7 ppm (s,4H,Ar$CH_2$NH); 6,1-6,7 ppm (m,6H,ArH); 7,4 ppm (d,2H,ArH); 8,2 ppm (d,2H,ArH)

N,N'-Bis[Methyl-2-pyrrolyl]-2-(4'-aminobenzyl)malonsäurediamid[27]

Diese Verbindung wird entsprechend der Verbindung [6] dargestellt.
Ausbeute: 75%
$^1$H-NMR-Daten in DMSO/TMS
3,1 ppm (d,2H,$CH_2$Ar); 3,4 ppm (t,1H,CH); 3,7 ppm (s,4H,Ar$CH_2$NH); 6,2-6,9 ppm (m,10H,ArH)

N,N'-Bis[Methyl-2-pyrrolyl]-2-(4'-isithiocyanatobenzyl)malonsäurediamid[25]

Diese Verbindung wird entsprechend der Verbindung [7] dargestellt.
Ausbeute: 69%
$^1$H-NMR-Daten in DMSO/TMS
3,1 ppm (d,2H,$CH_2$Ar); 3,4 ppm (t,1H,CH); 3,7 ppm (s,4H,Ar$CH_2$NH); 6,2-7,0 ppm (m,10H,ArH)

**Beispiel 5**

N,N'-Bis[(2-(benzylthio)benzyl]-2-methyl-2-(4'-nitrobenzyl)malonsäurediamid [29]

Diese Verbindung wird entsprechend der Verbindung [4] dargestellt.
Ausbeute: 72%
$^1$H-NMR-Daten in $CDCl_3$/TMS
1,8 ppm (s,3H,Me); 3,1 ppm (d,2H,$CH_2$Ar); 4,1 ppm (s,4H,Ar$CH_2$S); 4,3 ppm (s,4H,Ar$CH_2$NH); 7,1-7,6 ppm (m,18H,ArH); 8,3 ppm (m,4H,ArH).

N,N'-Bis(2-mercaptobenzyl)-2-methyl-2-(4'-nitrobenzyl)malonsäurediamid [30]

Zu einer auf -50 °C gekühlten Lösung von 5,1 g [29] in 50 ml THF und 100 ml flüssigem Ammoniak werden 3,5 g Natrium vorsichtig zugegeben und rührt 4 Stunden. Überschüssiges Natrium wird vorsichtig mit Ammoniumchlorid zerstört und läßt anschließend langsam auf Raumtemperatur erwärmen. Der Rückstand wird in Wasser aufgenommen und mehrmals mit Dichlormethan extrahiert, die organische Phase getrocknet und eingeengt.
Ausbeute: 59%
$^1$H-NMR-Daten in $CDCl_3$/TMS
1,5 ppm (s,3H,Me); 3,1 ppm (d,2H,$CH_2$Ar); 4,1 ppm (s,4H,Ar$CH_2$NH); 7,0-7,5 ppm (m,10H,ArH); 8,2 ppm (d,2H,ArH).

N,N'-Bis(2-mercaptobenzyl)-2-methyl-2-(4'-aminobenzyl)malonsäurediamid [31]

Diese Verbindung wird entsprechend der Verbindung [6] dargestellt.
Ausbeute: 69%
$^1$H-NMR-Daten in $CDCl_3$/TMS
1,4 ppm (s,3H,Me); 3,0 ppm (d,2H,$CH_2$Ar); 4,1 ppm (s,4H,Ar$CH_2$NH); 7,0-7,4 ppm (m,10H,ArH);

N,N'-Bis(2-mercaptobenzyl)-2-methyl-2-(4'-isothiocyanatobenzyl)malonsäurediamid [32]

Diese Verbindung wird entsprechend der Verbindung [7] dargestellt.

Ausbeute: 49%
$^1$H-NMR-Daten in DMSO/TMS
1,4 ppm (s,3H,Me); 3,0 ppm (d,2H,$CH_2$Ar); 4,1 ppm (s,4H,Ar$CH_2$NH); 7,0-7,5 ppm (m,10H,ArH);

**Beispiel 6**

2-Methyl-2-(2-propinyl)-malonsäurediethylester [33]

Diese Verbindung wird entsprechend der Verbindung [1] dargestellt.
Ausbeute: 66%
$^1$H-NMR-Daten in $CDCl_3$/TMS
1,5 ppm (s,3H,Me); 2,0 ppm (t,1H,CCH); 2,8 ppm (m,2H,$CH_2$); 3,8 ppm (s,6H, COOMe)

2-Methyl-2-(2-propinyl)-malonsäure [34]

Diese Verbindung wird entsprechend der Verbindung [2] dargestellt.
Ausbeute: 81%
$^1$H-NMR-Daten in $CDCl_3$/TMS
1,4 ppm (s,3H,Me); 2,6 ppm (d,2H,$CH_2$); 2,9 ppm (t,1H,CCH);

2-Methyl-2-(2-propinyl)-malonsäurechlorid [35]

Diese Verbindung wird entsprechend der Verbindung [3] dargestellt.
Ausbeute: 91%

N,N'-Bis[(2-benzylthio)benzyl -2-methyl-2-(2-propinyl)malonsäurediamid [36]

Diese Verbindung wird entsprechend der Verbindung [4] dargestellt.
Ausbeute: 67%
$^1$H-NMR-Daten in $CDCl_3$/TMS
1,5 ppm (s,3H,$CH_3$); 2,0 ppm (t,1H,CCH); 2,8 ppm (d,2H,$CH_2$CC); 4,1 ppm (s,4H,Ar$CH_2$S); 4,2 ppm (s,4H,Ar$CH_2$NH); 7,1-7,6 ppm (m,18H,ArH).

N,N'-Bis[(2-mercaptobenzyl)] -2-methyl-2-(2-propinyl)malonsäurediamid [37]

Diese Verbindung wird entsprechend der Verbindung [30] dargestellt.
Ausbeute: 70%
$^1$H-NMR-Daten in $CDCl_3$/TMS
1,5 ppm (s,3H,$CH_3$); 2,0 ppm (t,1H,CCH); 2,8 ppm (d,2H,$CH_2$CC); 4,0 ppm (s,4H,Ar$CH_2$S); 7,0-7,5 ppm (m,8H,ArH).

**Beispiel 7**

N,N'-Bis[(2-benzylthio-3-pyridyl)methyl]-2-methyl-2-(4'-nitrobenzyl)malonsäurediamid [38]

Diese Verbindung wird entsprechend der Verbindung [4] dargestellt.
Ausbeute: 60%
$^1$H-NMR-Daten in $CDCl_3$/TMS
1,2 ppm (s,3H,Me); 3,5 ppm (s,2H,$CH_2$Ar); 4,3 ppm (s,4H,Ar$CH_2$S); 4,4 ppm (s,4H,Ar$CH_2$NH); 7,1-7,5 ppm (m,16H,ArH); 8,3-8,5 ppm (m,4H,ArH)

N,N'-Bis[(2'-thio-3'-pyridyl)methyl]-2-methyl-2-(4''-nitrobenzyl)malonsäurediamid [39]

Diese Verbindung wird entsprechend der Verbindung [30] dargestellt.
Ausbeute: 61%
$^1$H-NMR-Daten in DMSO/TMS
1,2 ppm (s,3H,Me); 3,4 ppm (s,2H,$CH_2$Ar); 4,3 ppm (s,4H,Ar$CH_2$NH); 7,1-7,5 ppm (m,6H,ArH); 8,3-8,5 ppm (m,4H,ArH).

N,N'-Bis[(2'-thio-3'-pyridyl)methyl]-2-methyl-2-(4''-aminobenzyl)malonsäurediamid [40]

Diese Verbindung wird entsprechend der Verbindung [6] dargestellt.
Ausbeute: 66%
$^1$H-NMR-Daten in DMSO/TMS
1,2 ppm (s,3H,Me); 3,3 ppm (s,2H,$CH_2$Ar); 4,3 ppm (s,4H,Ar$CH_2$NH); 7,1-7,5 ppm (m,8H,ArH); 8,5 ppm (d,2H,ArH).

N,N'-Bis[(2'-thio-3'-pyridyl)methyl]-2-methyl-2-(4''-isothiocyanatobenzyl)malonsäurediamid [41]

Diese Verbindung wird entsprechend der Verbindung [7] dargestellt.
Ausbeute: 82%
$^1$H-NMR-Daten in DMSO/TMS
1,3 ppm (s,3H,Me); 3,3 ppm (s,2H,$CH_2$Ar); 4,3 ppm (s,4H,Ar$CH_2$NH); 7,2-7,5 ppm (m,8H,ArH); 8,5 ppm (d,2H,ArH).

**Beispiel 8**

N,N'-Bis(2-methoxybenzyl)-2-methyl-2-(2-propinyl)malonsäurediamid [42]

Diese Verbindung wird entsprechend der Verbindung [4] dargestellt.
Ausbeute: 76%
$^1$H-NMR-Daten in $CDCl_3$/TMS
1,5 ppm (s,3H,$CH_3$); 2,0 ppm (t,1H,CCH); 2,8 ppm (m,2H,$CH_2$); 3,8 ppm (s,6H,$CH_3$OAr); 4,4ppm (d,4H,Ar$CH_2$NH); 6,8-7,4 ppm (m,8H,ArH).

**Beispiel 8a**

2-Methyl-2-(prop-1-inyl)-1,3-bis-(2-hydroxy-benzylamido)-propan [43]

200 mg 2-Methyl-2-(prop-1-inyl)-1,3-bis-(2-methoxy-benzylamido)-propan [42] wurden in 4 ml getrocknetem Dichlormethan gelöst. Unter Stickstoff wurden 3.8 ml einer einmolaren Lösung von Bortribromid in Dichlormethan zugetropft. Die Mischung wurde 4 Stunden auf 40 ° C erwärmt. Nach Abkühlen hydrolysierte man, entfernte nach Trennung der Phasen und zweimaligem Nachextrahieren das Lösungsmittel und chromatographierte an Kieselgel mit Dichlormethan/Aceton (0-30%). Man erhieltein zähes Öl.
Ausbeute: 70 %
$^1$H-NMR-Daten in $CDCl_3$/TMS
1.53 ppm (s,3H,Me); 1.97 ppm (t,1H,CCH); 2.77 ppm (d,2H, $CH_2$-CC); 4.85 ppm (m,4H, Ar$CH_2$NCO); 6.80-7.30 ppm (m,8H,ArH); 8.90 ppm (s,2H,OH)

**Beispiel 9**

17$\beta$-Hydroxy-17a-[6,6-bis-(N,N'-{o-methoxy}-benzyl-amino-carbonyl)-hept-1-(E)-en-3-in]-3-methoxy-1,3,5-östratrien [44]

40 mg (E)-17$\beta$-Hydroxy-17$\alpha$-(2-iodovinyl)-3-methoxy-1,3,5-östratrien und 48 mg des Komplexbildners [42] wurden zusammen mit 3.2 mg Bis-(triphenylphosphin)-palladiumdichlorid und 2.6 mg Kupfer(I)iodid in 1 ml Toluol (p.a.) gelöst, mit 22 mg Isopropylamin versetzt und unter Stickstoffbegasung 3 Stunden bei 50 ° C gerührt. Nach dieser Zeit ließ man abkühlen, entfernte das Lösemittel und reinigte per Flüssigchromatographie an Kieselgel mit Hexan/Essigsäureethylester (1:1) als Laufmittel.
Ausbeute: 65%
$^1$H-NMR-Daten in $CDCl_3$/TMS
0.92 ppm (s,3H,Me[18]); 1.20-3.00 ppm (m,15H,Steroid); 1.55 ppm (s,3H,Me); 2.85 ppm (d,2H,$CH_2$-CC); 3.78 ppm (s,3H,Steroid-OMe); 3.85 ppm (s,6H, Ar-OMe); 4.45 ppm (m,4H,Ar$CH_2$NCO); 5.55 und 6.23 ppm (AB,2H,CH = CH); 6.60 - 7.35 ppm (m,11H,Ar);

17$\beta$-Hydroxy-17$\alpha$-[6,6-bis-(N,N'-{o-hydroxy}-benzyl-amino-carbonyl)-hept-1-(E)-en-3-in]-3-methoxy-1,3,5-östratrien [45]

7.14 mg (E)-17β-Hydroxy-17α-(2-iodovinyl)-3-methoxy-1,3,5-östratrien und 8 mg des Komplexbildners [43] wurden zusammen mit 0.56 mg Bis-(triphenylphosphin)-palladiumdichlorid und 0.46 mg Kupfer(I)iodid in 0.5 ml Toluol (p.a.) gelöst, mit 4 mg Isopropylamin versetzt und unter Stickstoffbegasung 3 Stunden bei 50°C gerührt. Nach dieser Zeit ließ man abkühlen, entfernte das Lösemittel und reinigte die gesamte Menge über 5 HPTLC Fertigplatten (Merck, Kieselgel 60, 20 x 20 cm mit Konzentrierungszone) mit Hexan/Essigsäureethylester (1:1) als Laufmittel.
Ausbeute: 60 %
$^1$H-NMR-Daten in $CDCl_3$/TMS
0.93 ppm (s,3H,Me[18]); 1.20-3.00 ppm (m,15H,Steroid); 1.55 ppm (s,3H,Me); 2.88 ppm (d,2H,$CH_2$-CC); 3.78 ppm (s,3H,OMe); 4.37 ppm (m,4H,$ArCH_2NCO$); 5.40 und 6.20 ppm (AB,2H,CH=CH); 6.60 - 7.30 ppm (m,11H,Ar); 8.50 ppm (m,2H,OH)

3,17β-Dihydroxy-17α-[6,6-bis-(N,N'-{o-hydroxy}-benzyl-amino-carbonyl)-hept-1-(E)-en-3-in]-1,3,5-östratrien [46]

8.63 mg (E)-3,17β-Dihydroxy-17α-(iodovinyl)-1,3,5-östratrien und 15 mg des Komplexbildners [43] wurden zusammen mit 0.7 mg Bis-(triphenylphosphin)-palladiumdichlorid und 0.58 mg Kupfer(I)iodid in 0.6 ml Toluol (p.a.) gelöst, mit 5 mg Isopropylamin versetzt und unter Stickstoffbegasung 12 Stunden bei 60°C gerührt. Man entfernte das Lösemittel und reinigte die gesamte Menge über 5 HPTLC Fertigplatten (Merck, Kieselgel 60, 20 x 20 cm mit Konzentrierungszone) mit Hexan/Essigsäureethylester (3:7) als Laufmittel.
Ausbeute: 35%
$^1$H-NMR-Daten in $CDCl_3$/TMS
0.93 ppm (s,3H,Me[18]); 1.20-3.00 ppm (m,15H,Steroid); 1.55 ppm (s,3H,Me); 2.88 ppm (d,2H,$CH_2$-CC); 4.36 ppm (m,4H,$ArCH_2NCO$); 5.40 und 6.20 ppm (AB,2H,CH=CH); 6.55 - 7.25 ppm (m,11H,Ar); 8.50 ppm (m,2H,OH)

**Beispiel 10**

17β-Hydroxy-17α-[6,6-bis(N,N'-{o-methoxy}-benzyl-amino-carbonyl)hept-1-(Z)-en-3-in]-4-östren-3-on [47]

50 mg (E)-17β-Hydroxy-17α-(iodovinyl)-4-östren-3-on und 35 mg des Komplexbildners [42] wurden zusammen mit 24 mg Bis-(triphenylphosphin)-palladiumdichlorid und 12.5 mg Kupfer(I)iodid in 2 ml Toluol (p.a.) gelöst, mit 23 mg Isopropylamin versetzt und unter Stickstoff 8 Stunden bei 60°C gerührt. Nach weiteren 60 Stunden Rühren bei Raumtemperatur entfernte man das Lösemittel und reinigte die gesamte Menge über 10 HPTLC Fertigplatten (Merck, Kieselgel 60, 20 x 20 cm mit Konzentrierungszone) mit Hexan/Essigsäureethylester (3:7) als Laufmittel.
Ausbeute: 25%
$^1$H-NMR-Daten in $CDCl_3$/TMS
1.00 ppm (s,3H,Me[18]); 1.20-2.60 ppm (m,20H,Steroid); 1.40 ppm (s,3H,Me); 3.23 ppm (AB,2H,$CH_2$-CC); 3.82 ppm (s,6H,OMe); 4.45 ppm (m,4H,$ArCH_2NCO$); 5.83 ppm (s,1H,C4-Steroid); 6.34 ppm (AB,2H,CH=CH); 6.80 und 7.20 ppm (m,8H,Ar);

17β-Hydroxy-17α-[6,6-bis(N,N'-{o-methoxy}-benzyl-amino-carbonyl)hept-1-(E)-en-3-in]-4-östren-3-on [48]

Diese Verbindung wurde entsprechend Verbindung [47] dargestellt.
Ausbeute: 33%
$^1$H-NMR-Daten in $CDCl_3$/TMS
1.00 ppm (s,3H,Me[18]); 1.20-2.60 ppm (m,20H,Steroid); 1.50 ppm (s,3H,Me); 2.92 ppm (m,2H,$CH_2$-CC); 3.83 ppm (s,6H,OMe); 4.42ppm (m,4H,$ArCH_2NCO$); 5.82 ppm (s,1H,C4-Steroid); 5.45 und 5.95 ppm (AB,2H,CH=CH); 6.85 und 7.20 ppm (m,8H,Ar);

3,3-Ethylendioxy-17β-hydroxy-17α-[6,6-bis(N,N'-{o-methoxy}-benzyl-amino-carbonyl)hept-1-(E)-en-3-in]-5-(6)-östren [49]

Diese Verbindung wurde entsprechend Verbindung [47] dargestellt.
Ausbeute: 31%
$^1$H-NMR-Daten in $CDCl_3$/TMS
0.90ppm (s,3H,Me[18]); 0.85-2.20 ppm (m,20H,Steroid); 1.55 ppm (s,3H,Me); 2.92ppm (m,2H,$CH_2$-CC); 3.85

ppm (s,6H,OMe); 3.95 ppm (m,4H,$OCH_2CH_2O$), 4.45 ppm (m,4H,$ArCH_2NCO$); 5.45 ppm (s,1H,C6-Steroid); 5.50 und 6.18 ppm (AB,2H,CH = CH); 6.90 und 7.20 ppm (m,8H,Ar);

17β-Hydroxy-17α-[6,6-bis(N,N'-{o-hydroxy}-benzyl-amino-carbonyl)hept-1-(Z)-en-3-in]-4-östren-3-on [50]

Diese Verbindung wurde entsprechend Verbindung [47] dargestellt.
Ausbeute: 12%
$^1$H-NMR-Daten in $CDCl_3$/TMS
1.00ppm (s,3H,Me[18]); 1.20-2.60 ppm (m,20H,Steroid); 1.40ppm (s,3H,Me); 3.26 ppm (AB,2H,$CH_2$-CC); 4.40 ppm (m,4H,$ArCH_2NCO$); 5.81 ppm (s,1H,C4-Steroid); 6.30 ppm (AB,2H,CH = CH); 6.80 und 7.20 ppm (m,8H,Ar); 8.50 ppm (m,2H,OH)

3,3-Ethylendioxy-17β-hydroxy-17α-[6,6-bis(N,N'-{o-hydroxy}-benzyl-amino-carbonyl)hept-1-(E)-en-3-in]-4-östren-3-on [51]

Diese Verbindung wurde entsprechend Verbindung [47] dargestellt.
Ausbeute: 18%

**Beispiel 11**

Kopplung und Markierung eines Isothiocyanat-haltigen Chelatbildners an Proteine

Am Beispiel von $F(ab)_2$-Fragmenten des monoklonalen Antikörpers 17-1A soll die Kopplung von Isothiocyanat-haltigen Tc-Chelatbildnern Verbindung [6] an Proteine beschrieben werden. Anstelle der Antikörper-Fragmente kann jedes andere Protein bzw. eine Aminogruppen-haltige Substanz verwendet werden.

Der monoklonale Antikörper 17-1A wird entsprechend literaturbekannter Methoden nach Applikation von $10^7$ der entsprechenden Hybridomazellen in den Bauchraum einer Balb/c-Maus und Aspiration der Ascitesflüssigkeit nach 7 - 10 Tagen gewonnen. Die Reinigung erfolgt nach ebenfalls literaturbekannten Methoden durch Ammoniumsulfatfällung und Affinitätschromatographie über Protein A-Sepharose. Der gereinigte Antikörper (10 mg/ml) wird bei pH 3,5 für 2 Std. mit 25 µg/ml Pepsin behandelt und anschließend mittels FPLC isoliert. Vor der Kopplung mit dem Chelatbildner werden die Fragmente bei 4° C für 12- 24 Std. gegen 0,1 M $KH_2PO_4$/0,1 M $NaHCO_3$, pH 8,5, dialysiert. Die Proteinkonzentration wird auf 10 mg/ml eingestellt. Ein 5facher molarer Überschuß des NCS-haltigen Chelatbildners [Beispiel 1] wird in möglichst wenig des gleichen Puffers gelöst und zur Proteinlösung hinzugegeben. Zur Konjugatbildung wird die Mischung für 3 Std. bei 37 ° C Inkubiert. Anschließend wird das Konjugat für 24 - 48 Std. mit mehrfachem Pufferwechsel gegen PBS (Phosphat-gepufferte Saline) dialysiert und die Proteinkonzentration danach nötigenfalls wieder auf 10 mg/ml eingestellt. Bis zur Markierung mit Tc-99m kann das Konjugat nach Sterilfiltration bei 4 ° C in säuregereinigten Glasgefäßen aufbewahrt werden.

Die Markierung von 1 mg des mit dem Chelatbildner [6] gekoppelten Antikörper-Fragments mit Tc-99m erfolgt durch Zugabe von 10 mCi Pertechnetatlösung ( = 1 - 2ml) und 100 µg Zinn-II-chlorid in einer Argongespülten Na-Pyrophosphat-Lösung (1mg/ml) oder durch Ligandenaustausch z.B. durch Zugabe der Lösung eines mit Pertechnetat versetzten kommerziell erhältlichen Glucoheptonat-Kits.

**Beispiel 12**

2-Methyl-2-[(3-tert. butyloxycarbonyl)methyl]-malonsäuredimethylester [52]

Zu einer Suspension von 600 mg Natriumhydrid (0,025 mol) in 25 ml wasserfreiem DMF wird unter Rühren die Lösung von 4,36 g Methylmalonsäuredimethylester (0,025 mol) in 5 ml wasserfreiem DMF langsam zugetropft. Nach beendeter Zugabe wird noch 30 min gerührt, anschließend die Lösung von 5,85 g Bromessigsäure-t-butylester (0,030 mmol) in 10 ml DMF zugetropft und noch weitere 2 h bei RT gerührt-(DC-Kontrolle). Nach vollständiger Umsetzung wird auf 300 ml Eiswasser gegossen, neutralisiert und mit einem geeigneten Lösungsmittel extrahiert. Nach Trocknen über Natriumsulfat wird das Lösungsmittel abgezogen und der Rückstand destilliert.
Ausbeute:70%
$^1$H-NMR-Daten in $CDCl_3$/TMS
1,4 ppm (s,9H,Me); 1,5 ppm (s,3H,Me); 2,8 ppm (s,2H,$CH_2COO$); 3,8 ppm (t,6H,Me).

2-Methyl-2-[(3-tert. butyloxycarbonyl)methyl]-malonsäure[53]

Diese Verbindung wurde entsprechend Verbindung [2] dargestellt.
Ausbeute: 53%
$^1$H-NMR-Daten in DMSO/TMS
1,4 ppm (s,9H,Me); 1,5 ppm (s,3H,Me); 2,6 ppm (s,2H,$CH_2$COO).

2-Methyl-2-[(3-tert. butyloxycarbonyl)methyl]-malonsäuredichlorid[54]

Diese Verbindung wurde entsprechend Verbindung [3] dargestellt.
Ausbeute: 72%
$^1$H-NMR-Daten in $CDCl_3$/TMS
1,4 ppm (s,9H,Me); 1,5 ppm (s,3H,Me); 2,7 ppm (s,2H,$CH_2$COO).

N,N'-Bis[(2-p-methoxybenzylthio-3-pyridyl)methyl]-2-methyl-2-[2-(tert.-butyloxycarbonyl)methyl]-malonsäurediamid [55]

Diese Verbindung wurde entsprechend Verbindung [4] dargestellt.
Ausbeute: 59%
$^1$H-NMR-Daten in DMSO/TMS
1,4 ppm(s,9H,Me); 1,5 ppm (s,3H,Me); 2,7 ppm (s,2H,$CH_2$COO), 3,7 ppm (s,6H,OMe); 4,3 ppm (m,8H,Ar$CH_2$S, Ar$CH_2$NH); 6,8 ppm (d,2H,ArH); 7,1ppm (dd,2H,ArH); 7,3 ppm (m,4H,ArH); 7,6 ppm (d,2H,ArH); 8,4 ppm (t,2H,ArH).

N,N'-Bis[(2-mercapto-3-pyridyl)methyl]-2-methyl-2-(2-carboxymethyl)malonsäurediamid [56]

Zu 10 ml Trifluoressigsäure werden bei Raumtemperatur 580 mg der Verbindung [55] gegeben und zum Sieden erhitzt. Nach beendeter Reaktion wird die Trifluoressigsäure im Vakuum abgezogen, der Rückstand in Chloroform aufgenommen, mit Wasser gewaschen, getrocknet und eingeengt.
Ausbeute: 49%
$^1$H-NMR-Daten in DMSO/TMS
1,5 ppm (s,3H,Me); 2,6 ppm (s,2H,$CH_2$COO), 4,2 ppm (m,4H,Ar$CH_2$NH); 6,8 ppm (d,2H,ArH); 7,1 ppm (dd,2H,ArH); 7,3 ppm (m,4H,ArH); 7,6 ppm (d,2H,ArH); 8,4 ppm (t,2H,ArH).

**Beispiel 12a**

N,N'-Bis[(2-p-methoxybenzylthio-3-pyridyl)methyl]-2-methyl-2-(2-carboxymethyl)-malonsäurediamid [57]

Zu 10 ml Trifluoressigsäure werden bei -10°C 1,2 g der Verbindung [55] gegeben und bei 0°C gerührt. Nach beendeter Reaktion wird die Trifluoressigsäure im Vakuum abgezogen, der Rückstand in Chloroform aufgenommen, mit Wasser gewaschen, getrocknet und eingeengt und auf Kieselgel chromatographiert.
Ausbeute: 59%
$^1$H-NMR-Daten in DMSO/TMS
1,5 ppm (s,3H,Me); 2,6ppm (s,2H,$CH_2$COO); 3,7 ppm (s,6H,OMe); 4,3 ppm (m,8H,Ar$CH_2$S, Ar$CH_2$NH); 6,8 ppm (d,2H,ArH); 7,1 ppm (dd,2H,ArH); 7,3 ppm (m,4H,ArH); 7,6 ppm (d,2H,ArH); 8,4 ppm (t,2H,ArH).

**Patentansprüche**

**1.** Verbindungen der allgemeinen Formel I

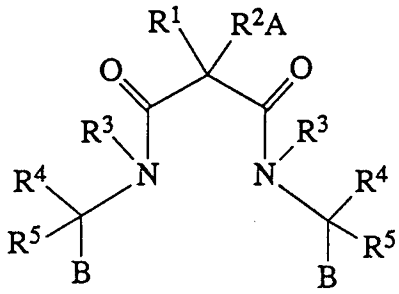

(I)

worin $R^1$ für Wasserstoff oder einen gegebenenfalls mit ein oder zwei Hydroxylgruppen substituierten $C_{1-6}$-Alkylrest,

$R^2$ für einen gegebenenfalls mit einer Hydroxylgruppe substituierten $C_{1-6}$-Alkylenrest,

$R^3$ für ein Wasserstoffatom oder einen $C_{1-6}$-Alkylrest,

$R^4$ für ein Wasserstoffatom, einen gegebenenfalls mit eine Hydroxylgruppe substituierten $C_{1-6}$-Alkylrest bzw. für die unter $R^x$ angegebene Bedeutung steht,

$R^5$ für ein Wasserstoffatom oder einen $C_{1-6}$-Alkylrest steht,

B für einen Pyrrolyl-, einen substituierten Phenylrest der Formel II oder einen substituierten Pyridinrest der Formel III

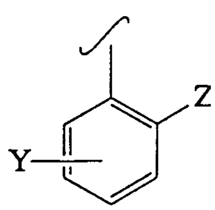

(II)

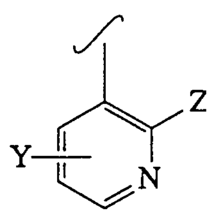

(III)

worin Z eine Hydroxyl-, Amino- oder eine Mercaptogruppe und Y ein Wasserstoffatom, einen Carboxy- oder einen Sulfonylrest bedeuten

oder für einen Nitrosomethylrest der Formel IV

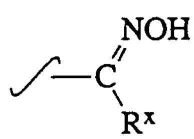

(IV)

steht,

worin $R^x$ einen $C_{1-6}$-Alkylrest, der gegebenenfalls zusammen mit $R^4$ über eine Trimethylen- oder Tetramethylengruppe zu einem 5- bzw. 6-Ring cyclisiert ist, bedeutet

und A für einen Amino-, einen Mercapto-, einen Hydrazino-, einen Carbohydrazino-, einen Carboxy-, einen $C_{2-6}$-Alkinyl- oder -Alkenyl-, einen Oxiranyl-, einen fluorierten Phenoxycarbonyl-, einen gegebenenfalls mit einem Natriumsulfatrest substituierten Succinimidoxycarbonyl-, einen Aminophenyl- oder Isothiocyanatophenylrest steht,

wobei die Phenylgruppe gegebenfalls zusätzlich durch einen Carboxy-, einen Chlorsulfonyl- oder einen Sulfonsäurerest substituiert sein kann

oder eine - mit Hilfe der genannten funktionellen Gruppe gegebenenfalls über einen bifunktionellen Linkerrest L gebundene - sich selektiv in Läsionen oder bestimmten Geweben anreichernde Verbindung T enthält,

wobei T für monoklonale Antikörper oder deren Fragmente, Hormone, Enzyme, Liganden für Zellmembranrezeptoren, Steroide, Neurotransmitter, Lipide, Saccharide, Aminosäuren und Oligopeptide, Biotin, sowie Radiosensitizer, wie z.B. Misonidazol steht,

sowie deren Komplexe mit - zur Diagnostik und Tumortherapie geeigneten - radioaktiven Metallionen, sowie deren Salze mit anorganischen und organischen Säuren und Basen.

2. Verbindungen nach Anspruch 1, **dadurch gekennzeichnet,** daß der in A gegebenenfalls enthaltene Rest L aus bifunktionellen Linker wie z.B.

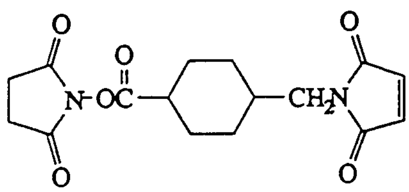

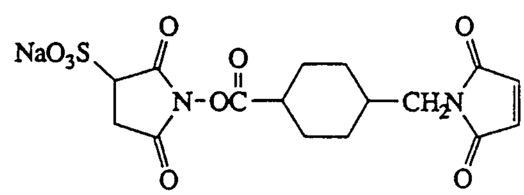

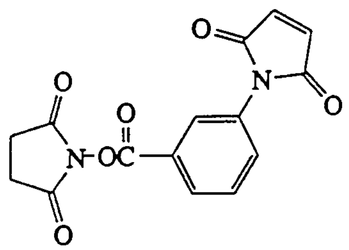

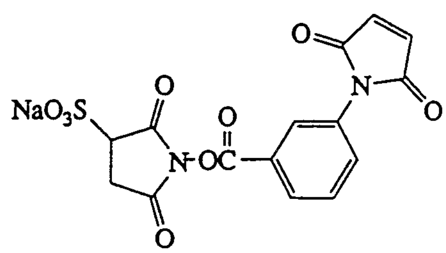

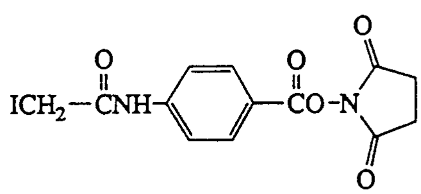

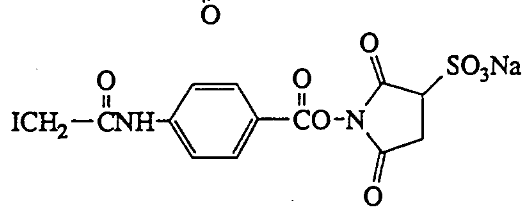

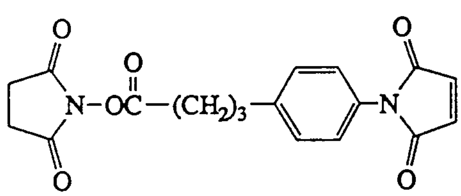

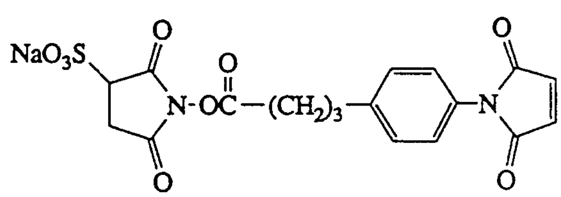

entstanden ist.

3. Verbindungen nach Anspruch 1, **dadurch gekennzeichnet,** daß T für monoklonale Antikörper, deren Fragmente, Biotin oder Misonidazol steht.

4. Metallchelate nach Anspruch 1 mit koordinativ gebundenen, radioaktiven Ionen von Tc, Re, Cu, Co, Ga, Y und In, bevorzugt Tc und Re.

5. Verwendung der Chelate gemäß Anspruch 4 für die In-vivo-Diagnostik und Tumortherapie.

**6.** Pharmazeutische Mittel enthaltend mindestens ein Chelat gemäß Anspruch 1, gegebenenfalls mit den in der Galenik üblichen Zusätzen.

**7.** Verfahren zur Herstellung von Verbindungen der allgemeinen Formel I

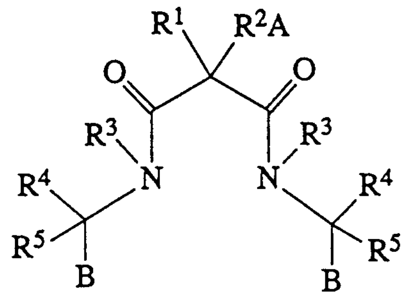

(I)

worin $R^1$ für Wasserstoff oder einen gegebenenfalls mit ein oder zwei Hydroxylgruppen substituierten $C_{1-6}$-Alkylrest,

$R^2$ für einen gegebenenfalls mit einer Hydroxylgruppe substituierten $C_{1-6}$-Alkylenrest,

$R^3$ für ein Wasserstoffatom oder einen $C_{1-6}$-Alkylrest,

$R^4$ für ein Wasserstoffatom, einen gegebenefalls mit einer Hydroxylgruppe substituierten $C_{1-6}$-Alkylrest bzw. für die unter $R^x$ angegebene Bedeutung steht,

$R^5$ für ein Wasserstoffatom oder einen $C_{1-6}$-Alkylrest steht,

B für einen Pyrrolyl-, einen substituierten Phenylrest der Formel II oder einen substituierten Pyridinrest der Formel III

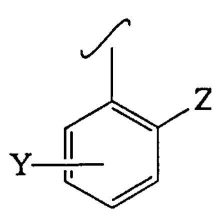

(II)

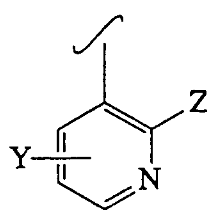

(III)

worin Z eine Hydroxyl-, Amino- oder eine Mercaptogruppe und Y ein Wasserstoffatom, einen Carboxy- oder einen Sulfonylrest bedeuten

oder für einen Nitrosomethylrest der Formel IV

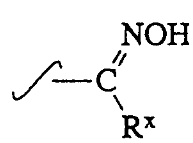

(IV)

steht,

worin $R^x$ einen $C_{1-6}$-Alkylrest, der gegebenenfalls zusammen mit $R^4$ über eine Trimethylen- oder Tetramethylengruppe zu einem 5- bzw. 6-Ring cyclisiert ist, bedeutet

und A für einen Amino-, einen Mercapto-, einen Hydrazino-, einen Carbohydrazido-, einen Carboxy-, einen $C_{2-6}$-Alkinyl- oder -Alkenyl-, einen Oxiranyl-, einen fluorierten Phenoxycarbonyl-, einen gegebenenfalls mit einem Natriumsulfatrest substituierten Succinimidoxycarbonyl-, einen Aminophenyl- oder Isothiocyanatophenylrest steht,

wobei die Phenylgruppe gegebenfalls zusätzlich durch einen Carboxy-, einen Chlorsulfonyl- oder einen Sulfonsäurerest substituiert sein kann

oder eine - mit Hilfe der genannten funktionellen Gruppe gegebenenfalls über einen bifunktionellen Linkerrest L gebundene - sich selektiv in Läsionen oder bestimmten Geweben anreichernde Verbindung T enthält,

wobei T für monoklonale Antikörper oder deren Fragmente, Hormone, Enzyme, Liganden für Zellmembranrezeptoren, Steroide, Neurotransmitter, Lipide, Saccharide, Aminosäuren und Oligopeptide, Biotin, sowie Radiosensitizer, wie z.B. Misonidazol steht,

sowie deren Komplexe mit - zur Diagnostik und Tumortherapie geeigneten - radioaktiven Metallionen, sowie deren Salze mit anorganischen und organischen Säuren und Basen,

**dadurch gekennzeichnet,** daß man

substituierte Malonsäurehalogenide, vorzugsweise Malonsäurechloride der allgemeinen Formel V,

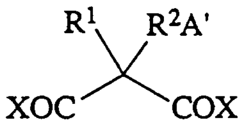 (V)

worin $R^1$, $R^2$ die genannten Bedeutungen haben, X für Halogen und A' für A oder einen in A umwandelbaren Rest steht

mit einem Amin der allgemeinen Formel VI

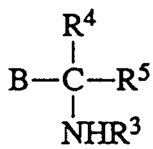 (VI)

in denen $R^3$, $R^4$, $R^5$ und B die oben genannten Bedeutungen haben und in B enthaltene funktionelle Gruppen gegebenenfalls in geschützter Form vorliegen,

in einem aprotischen Lösungsmittel, vorzugsweise Dichlormethan, bei Temperaturen von 0 - 180 °C, vorzugsweise bei Raumtemperatur, innerhalb von 2 bis 24 Stunden, vorzugsweise 4 Stunden, gegebenenfalls unter Zusatz von geeigneten Basen, z.B. Triethylamin, umsetzt und vorhandene Schutzgruppen abspaltet

undin den so erhaltenen Verbindungen die Gruppe A' gegebenenfalls zu A umwandelt - gegebenenfalls nach Schutz der freien Aminogruppen und der funktionellen Gruppen Z mit Schutzionen, z.B. als Cu-Komplex - generiert und anschließend gewünschtenfalls die so erhaltenen Verbindungen über diese funktionelle Gruppe an sich selektiv anreichernde Verbindungen T koppelt und den Substituenten B gewünschtenfalls mit dem jeweils gewünschten radioaktiven Isotop komplexiert, wobei vorher im Produkt gegebenenfalls vorhandene Schutzionen nach an sich literaturbekannten Methoden entfernt

werden und die Reihenfolge der Schritte Komplexierung mit radioaktiven Isotopen und Kopplung an T vertauscht werden kann.